# EUROPEAN PATENT APPLICATION

(11) **EP 1 566 202 A1**
(43) Date of publication of application: **24.08.2005**
(21) Application number: 04004071.9
(22) Date of filing: 23.02.2004
(51) Int. Cl.: A61P 19/02, A61K 31/557

(54) **Use of resistin antagonists in the treatment of rheumatoid arthritis**

(71) Applicant: Sahltech I Göteborg AB, 413 45 Göteborg (SE)
(72) Inventor: Smith, Ulf, 430 33 Fjäras (SE); Nagaev, Ivan, 413 26 Göteborg (SE); Bokarewa, Maria, 421 47 Västra Frölunda (SE); Tarkowski, Andrej, 413 26 Göteborg (SE)
(74) Representative: Becker Kurig Straus

(57) **Abstract**

The present invention relates to the use of an agent blocking the action of resistin for the treatment of Rheumatoid Arthritis. In particular, the present invention pertains to the use of resistin antagonists, such as anti-resistin antibodies, thiazolidinedione, oxazolidinedione, oxadiazolidinedione derivatives or mixtures thereof in the preparation of drugs for the prophylactic or therapeutic treatment of rheumatoid arthritis.

## Description

The present invention relates to the use of an agent blocking the action of resistin for the treatment of Rheumatoid Arthritis. In particular, the present invention pertains to the use of resistin antagonists, such as anti-resistin antibodies, thiazolidinedione, oxazolidinedione, oxadiazolidinedione derivatives or mixtures thereof in the preparation of drugs for the prophylactic or therapeutic treatment of rheumatoid arthritis.

Resistin, a 12.5-kDa protein belongs to a group of cysteine-rich secretory proteins originally described in mice as an adipocyte-derived polypeptide regulating adipocyte differentiation and modulating insulin-signaling pathway. Human resistin exhibits only 59% homology to its murine counterpart, with identity being highest in the cysteine-rich regions.

The limited protein sequence homology of resistin in humans and rodents and in addition, the diverse tissue distribution thereof led to discussions about divergent physiological functions of resistin in different species.

In mice, resistin expression in fat tissue and pre-adipocyte cell line suggested a direct implication to weight, glucose tolerance and insulin sensitivity of hepatocytes, skeletal muscle cells and adipocytes themselves. It was found that supplementation of mice with resistin was associated with the development of insulin resistance. Glitazones, a group of insulin-sensitizing antidiabetic drugs agonists of peroxisome proliferator-activated receptor (PPAR) gamma, were also found to be efficient suppressors of resistin levels.

The information about tissue expression and the physiological role of resistin in humans is controversial. Resistin is scanty expressed in fat tissues of lean subjects, while mononuclear leukocytes, spleen, and bone marrow cells seem to express high levels thereof. Low levels of resistin are expressed in lung tissues, resting endothelial cells and placental tissues. In contrast to the situation in mice, no difference in resistin expression in fat and muscle tissues was found in healthy and type 2 diabetes subjects (Furuhashi et al, Clin. Endocr. 2003, 59:507-10; Lee et al, J Clin. Endocr. Metabol. 2003, 88:4848-56) although circulating levels of resistin in these groups were different. Levels of resistin in humans were not related to other adipokines, adiponectine or leptin.

In general, the expression of resistin seems to be modulated by a variety of endocrine factors. In mouse adipocytes, resistin is induced by corticosteroids, prolactine, testosterone and growth hormone, while insulin, epinephrine, and somatotrophine seem to have an inhibitory effect (Banerjee & Lazar, J. Mol. Med. 2003, 81:218-26). Many of these substances belong to the nuclear hormone receptor family. On the transcriptional level resistin is repressed by PPAR-gamma, which binds to the human resistin gene promoter that has recently been identified (Patel et al, Biochem Biophys. Res. Commun. 2003, 300:472-6).

Investigations reported so far mainly focused on the role of resistin in fat and insulin metabolism. However, the fact that resistin is abundantly expressed in bone marrow and particularly in leukocytes, and that molecules of RELM family are found in inflamed tissues implied that resistin might have a role in other processes as well. Yet, reports published so far in this respect are quite contradictive.

It has been shown in subjects with type 2 diabetes that C-reactive protein is a predictive factor for resistin levels in circulation. Pro-inflammatory cytokines (IL-1, IL-6 and TNF-α) were able to induce expression of resistin in human PBMC (Kaser et al, Biochem Biophys Res Commun 2003, 309:286-90), while TNF-α was reported as a negative regulator of resistin expression in mouse adipocytes (Fasshauer et al, Biochem Biophys Res. Commun. 2001, 288:1027-31). Studies examining resistin expression as a part of endotoxin response showed that resistin mRNA expression is detectable in the early phase (Lu et al, FEBS Lett., 2002, 530:158-62) and undetectable following 24 h of endotoxin exposure (Rajala et al, Mol Endocrinol. 2002, 16:1920-30)

The sequence analysis of the resistin cDNA revealed the existence of a family of resistin-related molecules (RELM) which were found in inflammatory zone proteins (FIZZ), that were identified independently and all had each distinct tissue distribution. For example, FIZZ1/RELMa has been found in allergic pulmonary inflammation in mice, with no human analogue to FIZZ1 being identified so far. FIZZ2/RELMb, on the other hand is expressed predominantely in the small intestine and mucosal epithelial cells, while FIZZ3/resistin is expressed in white adipose tissue of rodents.

Since so far the actual physiological role of resistin still remained unresolved a problem of the present invention resides in further characterizing this protein and its natural task.

In the course of the present study it was surprisingly found that resistin is one of the major contributors of Rheumatoid Arthritis and its associated symptoms. Resistin accumulates in inflamed joints of patients with a level being significantly higher in synovial fluid than in the matched blood (22.1±3.2 versus 5.8±0.6 ng/ml, p<0.0001) and control synovial fluid samples, with the level of resistin in the joints correlating with the intensity of inflammation as defined by intra-articular WBC count and IL-6 level (r=0.66). It was shown that extracellular resistin had strong pro-inflammatory properties inducing synovitis when injected intra-articularly and triggering in vitro production of TNF-α, IL-6, and IL-1b of human peripheral blood monocytes (PBMC). Moreover, resistin has a governing position in the cytokine cascade, being able to respond to stimulation with TNF-α and its own. Pro-inflammatory properties of resistin were abrogated by NF-kappaB inhibitor indicating the importance of NF-kappaB signaling pathway for resistin induced inflammation.

Rheumatoid Arthritis is a chronic and recurrent systemic inflammatory disease primarily of the joints, usually poly-articular and characterized by inflammatory changes in the snyovial membranes and articular structures and by atrophy and rarefaction of the bones. Rheutmatoid arthritis alternates between remission and exacerbation and results in the damage and deformation of joints when left untreated. In late stages of the disease condition deformity and ankylosis may develop, eventually leading to a dysfunction of motor organs. Aspects of arthritis and joint damage causing Rheumatoid Arthritis, especially the pathological courses thereof, have been a focus of research works, leading to the present belief that Rheumatoid Arthritis seems to be induced by a number of factors including living environment and genetic background.

During the experiments leading to the present invention recombinant resistin has been injected into healthy knee joints, with the result that intra-articular accumulation of resistin showed strong pro-inflammatory properties. A single injection of recombinant mouse resistin in a dose 1 ng/knee (50 ng/ml) was sufficient to induce leukocyte infiltration and hyperplasia of synovia. This dose of resistin is well comparable with the amount of protein detected in synovial fluid during acute joint effusion in Rheumatoid Arthritis patients. Additionally, stimulation of synovial fluid leukocytes with resistin in vitro resulted in an increase of IL-6 and IL-1b mRNA.

It has also been shown that human PBMC recognize extracellular resistin and respond to this stimulation by the production of a broad spectrum of pro-inflammatory cytokines. Even more, resistin induced its own production in human PBMC providing a positive feedback. This spectrum of cytokines includes TNF-α having a dominant regulatory role in the cytokine cascade, and IL-6, and IL-1b. In contrast, only TNF-α treatment of PBMC was able to induce expression of resistin in vitro. Presence of an alternative way for cytokine activation in Rheumatoid Arthritis gives an explanation to for only partial effect of TNF- α inhibitors in certain group of patients and for the lack of effect following intra-articular injection of TNF-alpha inhibitors. Interestingly, the monocytic cell line THP-1, that lacks resistin gene expression was still capable to respond to resistin stimulation giving rise to cytokine release. This observation presents monocytes as target cells for resistin stimulation. Endothelial cells have been recently reported as resistin-sensitive cells, responding to resistin with upregulation of endothelin 1 and vascular cell adhesion molecule (Verma et al., Circulation, 2003, 108:736-40), indicating that resistin-sensitive cells may be distributed broader than resistin-producing. It was furthermore shown that NF-kappaB transcription pathway is important for the expression of resistin induced cytokines, since resistin stimulation induced translocation of NF-kappaB from cytoplasma to nucleus as detected by electrophoretic mobility shift assay. Moreover, a significant abrogation of resistin effects was achieved when PBMC were treated with NF-kappaB inhibitor, pathenolide, simultaneously with resistin stimulation.

Based on this finding the present invention was accomplished according to which the use of resistin antagonists is provided for preparing a medicament for treating rheumatoid arthritis.

In principle, for preventing the biological activity of resistin for the purpose indicated, the level of resistin in a patient, in particular in the joints, must be reduced. This may be achieved by either modulating the production of the polypeptide or by blocking the activity thereof.

Modulation of translation of the polypeptide may be achieved on the transcriptional and/or the translational level. On the transcriptional level a nucleotide antisense to the resistin mRNA or parts thereof may be utilized. The cDNA and gene sequence of resistin (Steppan et al, Nature 2001, 409:307-12; Holcomb et al, EMBO J, 2000, 19:4046-55) and the skilled person may devise nucleotides having the appropriate base content and length and may formulate the antisense-nucleotide based on his general knowledge. Alternatively, modulation of transcription may also be achieved by controlling cellular transcription as such, e.g. by PPAR-gamma (peroxisome proliferator-activated receptor), that binds to the resistin gene promotor, such preventing transcription of the gene.

According to another embodiment, also the activity of the resistin polypeptide may be blocked as such. This may be achieved by any compound binding to resistin and preventing the polypeptide from exerting its normal activity. Examples for such compounds are antibodies binding to resisting and blocking its activity, or thiazolidinedione, oxazolidinedione, oxadiazolidinedione derivatives, PPAR alfa agonists, such as leukotriene B4 and 8(S)-hydroxyeicosatetraenoic acid, LXR agonists, such as Acetyl-podocarpic dimer (APD), or mixtures thereof. Also glitazones, a group of insulin-sensitizing antidiabetic drugs agonists of peroxisome proliferator-activated receptor (PPAR) gamma, are efficient suppressors of resistin level.

The compounds used for decreasing the activity of resisitin may be formulated for administration by various different routes, such as topical and systemic, e. g. oral, parenteral, inhalable, and the like, by slow release, by sustained release and by a pump, and the like, and are administered in amounts which prevent or reduce the biological effects brought about by resistin in Rheumatoid Arthritis.

The formulations prepared are thus suitable for the prevention and alleviation of symptoms brought about by Rheumatoid arthritis. The compounds may be administered by themselves or in conjunction with other drugs and therapies, and in a preventative/prophylactive as well as a therapeutic course and may optionally co-formulated with carriers and other formulation ingredients as known in the art.

In general, the administration of the present agents may be conducted with an oral formulation having a liquid carrier such as a solution, suspension, an oil-in-water and water-in-oil emulsion, and/or may be administered as a powder, dragee, tablet, capsule, spray or aerosol. When administered as a topical formulation, the carrier may be selected from creams, gels, ointments, sprays, aerosols, patches, solutions, suspensions and emulsions.

When the formulation is injectable, the carrier may be selected from aqueous and alcoholic solutions and suspensions, oily solutions and suspensions and oil-in-water and water-in-oil emulsions, among others. When as a rectal formulation, it may be in the form of a suppository, when in the form of a transdermal formulation, the carrier is selected from aqueous and alcoholic solutions, oily solutions and suspensions and oil-in-water and water-in-oil emulsions, although others are also suitable. The transdermal formulation, may be aniontophoretic transdermal formulation, wherein the carrier is selected aqueous and alcoholic solutions, oily solutions and suspensions and oil-in-water and water-in-oil emulsions, and the formulation may also contain a transdermal transport promoting agent, of which many are known in the art. Also suitable for prolonged administration are implantable capsules or cartridges containing the formulation. In this case, the carrier may also be selected from aqueous and alcoholic solutions and suspensions, oily solutions and suspensions and oil in-water and water-in-oil emulsions, be a hydrophobic carrier, such as lipid vesicles or particles, e. g. liposomes made of N-(1-[2,3-dioleoxyloxi] propyl)-N, N,N-trimethyl-ammonium methylsulfate and/or other lipids, and microcrystals. For pulmonary applications, the formulation is preferably a respirable or inhalable formulation, e. g. in the form of an aerosol. For prolonged exposure of a target area, the oligo may be delivered through a localized implant, suppository, sublingual formulation, and the like, all of which are known in the art.

According to another embodiment, the present invention provides the use of resistin as a marker for determining the onset/development of Chronic Arthritis in an individual. According to the present finding the level of resistin in an individual, especially in the joints is increased, so that the said increase may be taken as a measure in the present method. Accordingly, the method comprises determining the level of resistin in synovial fluid sample obtained from an individual chronic arthritis and comparing the level with a control individual with traumatic/degenerative joint disease. Such a level is normally in the range of 3 to 15 ng/ml. In case an increase over the level indicated above has been determined, this may be taken as a sign of the development of Chronic Arthritis.

As a sample to be utilized here is e.g. synovial fluid.

In the figures,
**Figure 1** shows that resistin accumulates in the inflamed joints of patients with rheumatoid arthritis
**Figure 2** shows that intra-articular injection of resistin in knee joint of healthy mice induces arthritis. Histopathology of arthritic knee joint 4 days after injection of recombinant mouse resistin (1 ng/joint). Original magnification x10. SC, synovial cavity; C, cartilage; ST, synovial tissue; B, bone. Arrowheads indicate inflammatory cells in synovium. Infiltration of mononuclear cells in synovial tissue is apparent. Arrows indicate pannus formation and cartilage destruction.
**Figure 3** shows upregulation of cytokine gene expression in human PBMC following stimulation with resistin (A) 1000 ng/ml, (B) 100 ng/ml. Results are presented as an increase of gene expression in folds over the level of respective gene expression in non-stimulated cell culture.
**Figure 4** shows an increase of cytokine levels in supernatants following stimulation with resistin Human PBMC (2x106/ml) were stimulated with given concentration of recombinant human resistin, and supernatants were collected following 48 hours of stimulation.
**Figure 5** shows inhibition of resistin-induced expression of IL-6 following treatment with a) monoclonal anti-resistin antibodies (1-10 ∝g/ml); b) NF-kappaB inhibitor, parthenolide (10-25 µM). Results are presented as percent of IL-6 levels in resistin-stimulated PBMC cells without inhibitors.
**Figure 6 shows** expression of resistin and cytokine genes in human PBMC following stimulation with (A) TNF-alpha, 1-5 ng/ml, (B) IL-6, 1-5 ng/ml, (C) IL-1b, 1 ng/ml

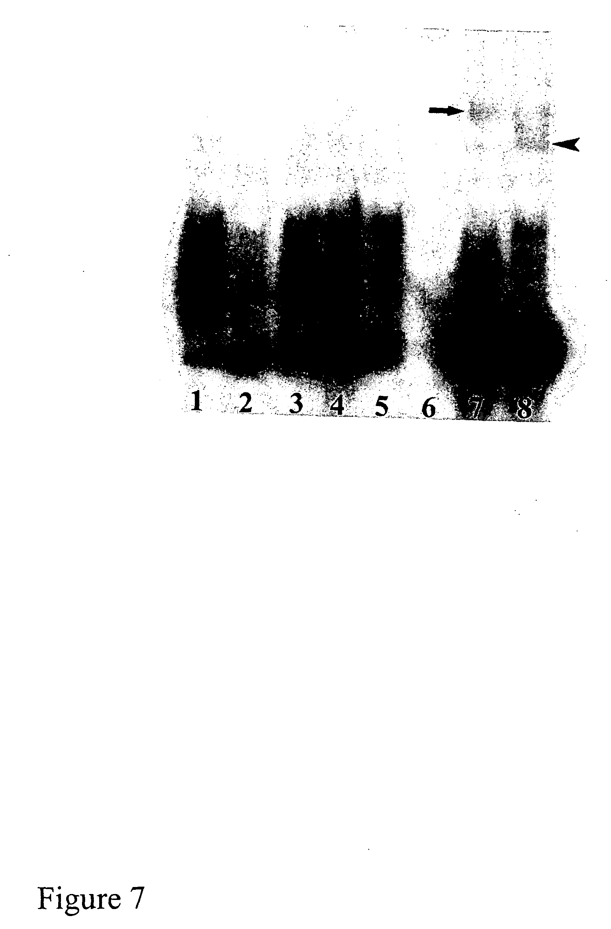

**Figure 7** shows binding of nuclear extracts from PBMC stimulated with resistin to NF-kappaB specific binding sites assessed by electrophoretic mobility shift assay (EMSA). Lanes 1. Binding of nuclear extracts stimulated with LPS (10 ∝g/ml) for 3 hours. Lanes 2-5. Binding of nuclear extracts from PBMC stimulated with recombinant human resistin 0-25-250-1000 ng/ml, for 3 hours. Lane 6. Nuclear extract as in lane 5 after pre-incubation with an excess of competitive binding unmarked NF-kappaB oligos. Lane 7. Shows a low mobility of the NF-kappaB complex with anti-p65 antibodies (marked with an arrow). Lane 8. Nuclear extract as in lane 5. Shows a low mobility of the NF-kappaB complex with anti-p65 antibodies (marked with an arrow). Lane 8. Nuclear extract as in lane 5. Shows a low mobility of the NF-kappaB complex with anti-p50 antibodies (marked with an arrow).

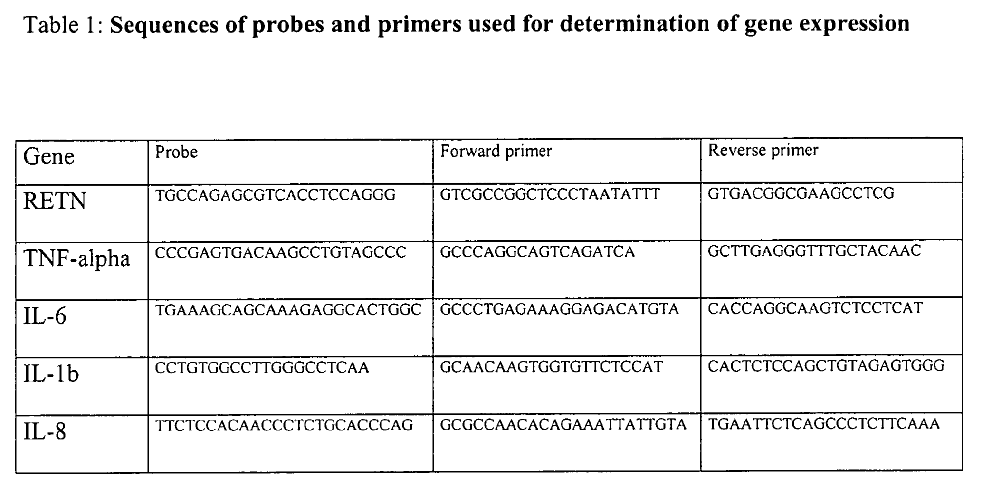

The following examples illustrate the invention without limiting it thereto.

### MATERIAL AND METHODS

### Patients

Plasma and synovial fluid samples were collected from 74 patients who attended the Rheumatology clinics, at Sahlgrenska University Hospital in Göteborg, for acute joint effusion. Rheumatoid Arthritis was diagnosed according to the American College of Rheumatology criteria 20. At the time of synovial fluid and blood sampling all the patients received non-steroidal anti-inflammatory drugs. Disease modifying anti-rheumatic drugs (DMARD) were used by 39 patients of which 24 used methotrexate (MTX). The remaining 35 patients had no DMARD treatment at the time of blood and synovial fluid sampling. Recent radiographs of the hands and feet were obtained for all the patients. Presence of bone erosions defined as the loss of cortical definition at the joint, was recorded in proximal interphalangeal, metacarpophalangeal, carpus, wrist and metatarsophalangeal joints. Presence of one erosion was sufficient to fulfill requirement of an erosive disease. Presence of RF of any of immunoglobulin isotypes was considered as positive.

### Collection and preparation of samples

Synovial fluid was obtained by arthrocentesis, aseptically aspirated and transmitted into the sodium citrate (0.129mol/l; pH 7.4) containing tubes. All synovial fluid samples were obtained from knee joints. Blood samples were simultaneously obtained from the cubital vein and directly transferred into sodium citrate medium. Blood samples from 34 healthy individuals (aged 18-6, mean 42±7 years) were used in the control group. Control synovial fluid was obtained from 21 patients (aged 36-88, mean 64±2) with non-inflammatory joint diseases (osteoarthritis 8 patients, chondrocalcinosis 2 patients, villonodular synovitis 1 patient, knee contusion 4, rupture of meniscus 4 patients, and cruciat ligament rupture 2 patients). Collected blood and synovial fluid samples were centrifuged at 800 g for 15 minutes, aliquoted, and stored frozen at -20oC until use.

### Laboratory parameters of disease activity

Serum levels of C-reactive protein (CRP) were measured with a standard nephelomitric assay with established normal range 0-5 mg/L. The erythrocyte sedimentation rate was measured by the Westergren method having normal range 0-20 mm/hour. White blood cell counts (WBC) in blood and in synovial fluid were performed using microcell counter F300 (Sysmex, Toa, Japan). Synovial fluid samples were treated with hyaluronidase before the cell count.

### Resistin levels

Resistin levels in patient samples and supernatants were determined by a sandwich ELISA (BioVendor Laboratory Medicine, Brno, Czech Republic). Briefly, 96-well polystyrene dishes (Nunc, Denmark) were coated with capture polyclonal anti-resistin antibodies and left overnight at room temperature. Following washing, plates were blocked and matched samples of plasma and synovial fluid were diluted 1:10 in BSA-PBS and introduced into the parallel strips. Biotin-labeled anti-resistin antibodies, streptavidin-horse raddish peroxidised conjugate and corresponding substrate were used for color development. Double wave length reading at 450 and 570 nm was used and the difference of absorbances was calculated. The obtained absorbance values were compared to serial dilution of recombinant human resistin and presented as ng/ml. Lowest detectable level was 1 ng/ml.

### Animal model of arthritis

Female NMRI mice, 6-8 weeks old, weighing 25-30g, were purchased from ALAB (Stockholm, Sweden). The mice were bred and housed in the animal facility of the Department of Rheumatology, University of Göteborg, under standard conditions of temperature and light, and fed laboratory chow and water *ad libidum.* Mouse recombinant resistin (PeproTech EC, London, U.K.) was injected intra-articularly into the right knee joint (0.1-100 ng/knee) in the total volume of 20 µl. Control mice obtained equivalent volume of mouse albumin (Sigma, St. Louise, MO) in PBS buffer. Three days after the joint injection, the mice were sacrificed by cervical dislocation and the right knee was removed for histological examination.

### Histological examination of joints

Histological examination of joints was done after routine fixation, decalcification, and paraffin embedding of the tissue. Tissue sections of the knee joints were cut and stained with hematoxylin and eosin. All the slides were coded and evaluated blindly by two investigators with respect to synovial hypertrophy, the inflammatory cell infiltration of synovia, pannus formation, and cartilage and subchondral bone destruction. Synovial hypertrophy was defined as synovial membrane thickness of more than two cell layers. Intensity of inflammatory cell infiltration of synovia was graded arbitrarily from 0 to 3.

### Cell preparation and culture conditions

Peripheral blood mononuclear cells (PBMC) were prepared from heparinazed blood of healthy individuals by separation on a Lymphoprep density gradient, washed, and resuspended in complete medium (Iscoves medium containing 1% L-glutamine, 5 × 10⁻⁵ M ®-mercapto ethanol, 50 mg/ml of gentamycin sulphate, and 10% heat inactivated fetal calf serum). Culturing of PBMC was carried out in 24-well plates in a humidified atmosphere of 5% CO2 at 37°C. Culturing of monocytic cell line THP-1 (American Type Culture Collection, Manassas, VA) was performed in 10-ml culture flasks (Nunclon) in RPMI 1649 medium supplemented with 10% FCS, 1% sodium pyruvate, gentamycin, and 2.5% Hepes in a humidified atmosphere of 5% CO₂ at 37°C. For the experiments, 4-days old cells were harvested, washed, and adjusted to 1 x 10⁶ cells/ml.

### Cell stimulation

Freshly isolated PBMC were resuspended to 2x10⁶/ml and stimulated with recombinant human resistin (endotoxin concentration less than 0.1 ng/∝g, PeproTech) at final concentration 10-1000 ng/ml. LPS (10 ng/ml) was used as a positive control in all the experiments. To assess specificity of resistin-induced effects, additional experiments were performed introducing monoclonal anti-resistin antibodies (R&D Systems, U.K.) in the cell culture prior to stimulation. Cell stimulation period differed depending on the read-out system used. For extracellular release of cytokines and resistin, supernatants were collected after 48 hours of stimulation. For assessment of gene expression, total mRNA was prepared 0, 3, and 24 hours of stimulation. Stimulation period for the analysis of transcription factors was 3 hours.

### Transfection procedure

Short inhibitory RNA (siRNA) sequence to human resistin was selected on the basis of mRNA sequence and using BLAST program dTdT marked 21 base oligonucleotides (targeting sequence 5'-AAUGAGAGGAUCCAGGAGGUC-3') were synthesized by MWG Oligo (Germany) and annealed to a duplex form. Sense stranded sequence was used as a negative control. siRNA were delivered to PBMC cells using Oligofectamine reagent (Invitrogen, Carlsbad, California). Before the transfection procedure cells were seeded in 96-well tissue culture plates and cultured overnight in Iscove's medium free of antibiotics and FCS. Transfection was assessed in Iscove's medium supplemented with 2.5% Hepes and 100 mg/ml CaCl2. For each well in a 96-well transfection, 0.6 ml Oligofectamine reagent was mixed with duplex siRNA (final concentration 60 and 120 pmol) and added to the washed cells. Following 4 h incubation at 37° C in a CO2 incubator, the transfection procedure was discontinued by adding growth Iscove's medium containing 3 time excess of FCS. At this time point cells were stimulated as required. Following 48 h of stimulation, cell cultures and supernatants were aseptically collected, centrifuged at 1000g for 5 min and kept frozen at -20°C until analyzed.

### Nuclear extracts preparation

Human PBMC (107) were stimulated with recombinant resistin (10-500 ng/ml) as described above. After 2 h, the stimulation was stopped with ice-cold PBS, cells were washed and resuspended in 2 ml hypotonic buffer (pH 7.9, containing 10 mM HEPES, 0.1 mM EDTA, 0.1 mM EGTA, 10 mM KCl, 0.75 mM spermidin, 0.15 mM spermin, 1 M dithiotreitol and proteinase inhibitors, Complete MiniTab, Boehringer) and homogenised. Following centrifugation at 14000g at 4° C for 10 min, the supernatant was removed and the pellet was resuspended in the ice-cold extraction buffer (pH 7.9, 20 mM HEPES, 0.42 M NaCl, 1 M EDTA, 1 mM EGTA, 25% glycerol, 1 M dithiotreitol and proteinase inhibitors). Extraction proceeded at 4° C under continuous rotation for 1 h. The supernatants containing nuclear extracts were collected after centrifugation 14000g for 1 h at 4° C. Protein concentration in the extracts was determined using Bradford reagent (Sigma). Nuclear extracts were aliquoted and stored at -70 °C until use.

### Electrophoretic mobility shift assay (EMSA)

EMSA was performed as described elsewhere (Bergquist et al, 2000) with minor modifications. The sequences for oligonucleotides used for the assay were as follows:

NFkappaB – sense 5'-GGCTCAAACAGGGGGCTTTCCCTCCTCAATAT-3',
antisense 5'-GGATATTGAGGAGG GAAAGCCCCCTGTTTGAG-3';

AP-1 – sense 5'-GGCTTCCTCCACATGAGATCATGGTTT TCT-3',
antisense 5'-GAGAAAACCATGATCTCATGTGGAGGAAG-3'.

Oligonucleotides were annealed at 56°C. The double stranded product was purified by elution from the electrophoretic gel. Double stranded oligonucleotides were labeled with [³²P]-deoxynucleotide (Amersham Pharmacia Biotech, Uppsala, Sweden) using Klenow polymerase (5 U/ml, Roche). Binding reactions were performed by incubation of nuclear extract protein (5 µg) in binding buffer (pH 7.9, 20 mM Tris-HCl, 30 mM NaCl, 5 µM EGTA, 5% glycerol) containing 0.2 mg/ml BSA, 5 µg poly (dI-dC), 1 mM dithiotreitol, and 1 µl of 32P-labelled double stranded oligonucleotides (0.1 pmol), at room temperature for 20 min. For competition studies, a 100-molar excess of unlabelled double stranded oligonucleotides was added to the reaction mixture and incubated for 20 min prior to the introduction of the 32P labeled probe. For supershift assays, antiserum to p65 and p50 subunits of NF-kappaB (Santa Cruz Biotechnology, CA) was incubated with nuclear extracts for 15 min at room temperature. Samples containing equal amount of protein were loaded directly onto 2.5% polyacrylamide gel prepared in Tris-borate-EDTA buffer (0.25x), and electrophoresis was performed at 200 V at room temperature. The gel was vacuum-dried and exposed to X-ray film for 48 h at -70° C.

### RNA isolation and RT-PCR assays

Total RNA from harvested cells was extracted by an RNAeasy kit (Qiagen, Inc.) and concentration was assessed spectrophotometrically at 260 nm. The gene expression was measured with TaqMan real time PCR (Applied Biosystems, Foster City, CA) as previously described (N&S, 2001). In short, total RNA extracts were treated with deoxyribonuclease I, and reverse transcribed with random nonamer primers and superscript II RnaseH- reverse transcriptase (life Technologies). After first strand cDNA synthesis, relative transcript cDNA levels were measured using oligonucleotide PCR primers and fluorogenic Taqman probes for test genes and 18S rRNA. Table 1 shows the sequences for the probes and primers used. For generation of the standard curve, serially diluted cDNA prepared from PBMC were used. Each TaqMan set span one or two exon joints in order to increase further both specificity of only RNA detection and efficiency of PCR reaction. Sequences were manually designed with using the PrimerExpress program (Applied Biosystems) to check them for secondary structure, melting temperature, GC content, primer to primer and/or to probe dimmers. The GenBank database was useful to avoid all known sites of oligonucleotide polymorphism and unspecific annealing of any nucleotide.

### Proliferation activity

Triplicate aliquots (200 ml) of THP-1 cell suspension were added to 96-well flatbottomed microtiter plates (Nunc, Copenhagen, Denmark) and incubated with test substance for 48 h. After that the cells were pulsed for 12 h with 1mCi of [3H]-thymidin (specific activity, 42 Ci/mmol; Amersham, Bucks, U.K.). Cells were collected into glass fiber filters. Thymidine incorporation in the filters was measured in beta-counter. The results were expressed as a stimulation index (cpm, mean±SD).

### Determination of IL-6 activity

The level of IL-6 in synovial fluid and supernatants was determined by a bioassay. The effect of test samples on proliferation of the IL-6 dependent cell line B13.29 [21] was assessed following 72 h of culturing. The results were analyzed by incorporation of [3H]-thymidine (Radiochemical Centre, Amersham, Bucks, U.K.) during the last 4 h of incubation at 37°C. Cells were collected into glass fiber filter. Proliferation in the presence of test samples was compared to the one induced by standard dilutions of recombinant IL-6 (Genzyme, Cambridge, MA).

### Determination of cytokine levels

Levels of TNF-α and IL-1β were assessed using an ELISA kit (R&D Systems) following manufacture's recommendations. Optical density of the tested samples was compared with the values obtained from serial dilution of respective recombinant human cytokine and expressed in pg/ml.

### Statistical analysis

The level of continuous variables was expressed as mean±SEM. Comparison between the matched blood and synovial fluid samples were analyzed by the paired t-test. For the evaluation of possible influence of radiological changes and ongoing treatment on the resistin levels, patient material was stratified accordingly. Difference between the groups was calculated separately employing the Mann-Whitney U test. Interrelation between parameters studied was calculated employing Spearman correlation coefficient. For all the statistical evaluation of the results, p-values below 0.05 were considered significant.

### RESULTS

### Increased levels of resistin in the inflamed joints

Resistin levels were assessed in the paired blood and synovial fluid samples of 74 patients with Rheumatoid Arthritis. Clinical and demographic data of the patient population and the control groups are presented in Table 2.

**Table 2.**

| **Clinical and demographic characteristics of patients with rheumatoid arthritis and of healthy controls** | | | | |
|---|---|---|---|---|
| | RA, erosive n=41 | RA. non-erosive n=33 | Controls | |
| | | | Blood n=34 | Syn.fluid n=21 |
| Age, years (range) | 64±2 (28-84) | 59±3 (24-83) | 42±7 (18-67) | 64±2 (33-88) |
| Sex, male/female | 14/27 | 8/23 | 12/22 | 11/10 |
| Duration of the disease, years | 12.6±1.5 | 5.7±1.1 | - | |
| Rheumatoid factor. +/- | 36/5 | 8/24 | n.a. | |
| Treatment with DMARD: | | | | |
| MTX n=24 | 18 | 6 | - | |
| Other DMARD:s n=15 | 9 | 7 | | |
| None n=34 | 14 | 20 | | |

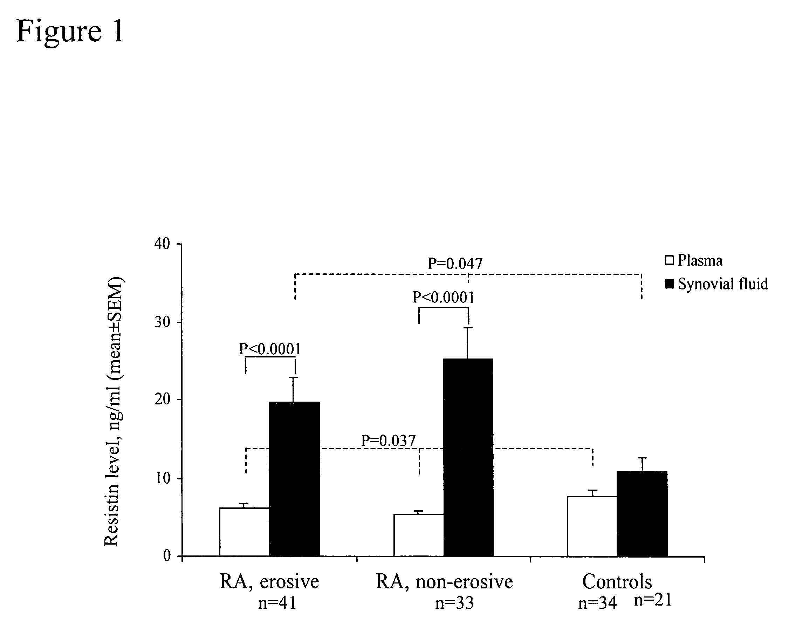

Synovial fluid samples showed a significant excess of extracellular resistin as compared to the matched blood (22.15±3.22 versus 5.77±0.57, p<0.0001; Figure 1). Resistin levels in synovial fluid samples of patients with Rheumatoid Arthritis were higher than in controls (22.15±3.22 versus 10.9±0.79, p=0.047), while blood samples revealed the opposite difference between Rheumatoid Arthritis and control group (5.77±0.57 versus 7.78±0.15, p=0.037). In synovial fluid, the level of resistin showed a significant correlation to WBC count (r=0.66) and to IL-6 levels (r=0.29). Resistin levels in blood was neither related to the duration of Rheumatoid Arthritis, age of the patients, nor to C-reactive protein levels and WBC count in blood. No difference in resistin levels in blood (6.14±0.79 versus 5.31±0.48, not significant) and synovial fluid (19.7±2.83 versus 25.2±5.07, not significant) was found between Rheumatoid Arthritis patients having erosive and nonerosive joint disease. Comparing Rheumatoid Arthritis patients receiving and non-receiving anti-rheumatic drugs, no difference in resistin levels was neither found in blood (5.57±0.76 versus 5.99±0.60, not significant) nor in synovial fluid samples (23.0±3.19 versus 21.21±5.40, not significant).

### Resistin induces inflammation when introduced intra-articularly

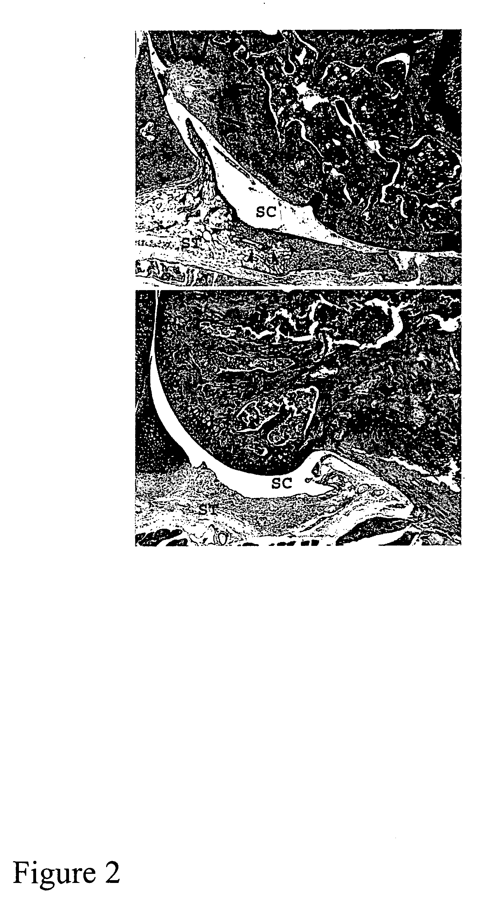

Recombinant mouse resistin was injected intra-articularly in the knee joint of healthy mice in the dose from 0.1-100 ng/knee. Control animals obtained mouse albumin 100 ng/knee. Histological analyses of the injected joints three days after the injection revealed signs of inflammation in the joint (hyperplasia of synovial tissues, leukocyte infiltration, pannus formation was observed in several occasions) (Figure 2). In the joints injected with control vehicle only 2 of 18 joints (11%) revealed signs of mild synovitis.

### Influence of resistin expression on inflammation

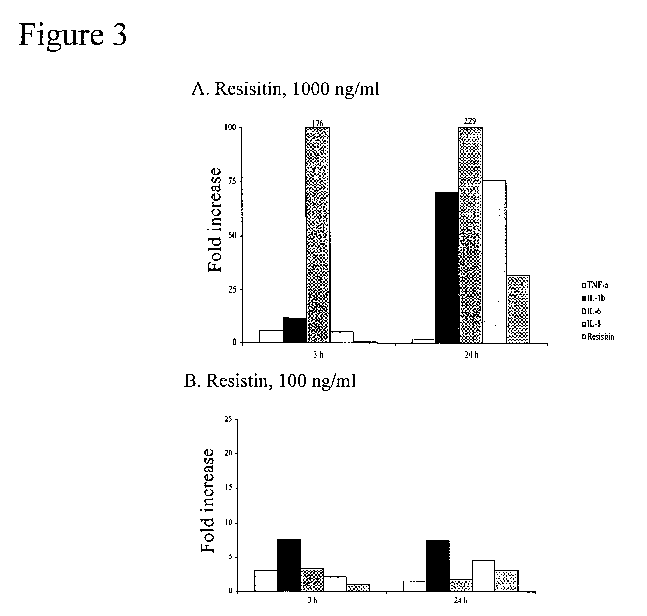

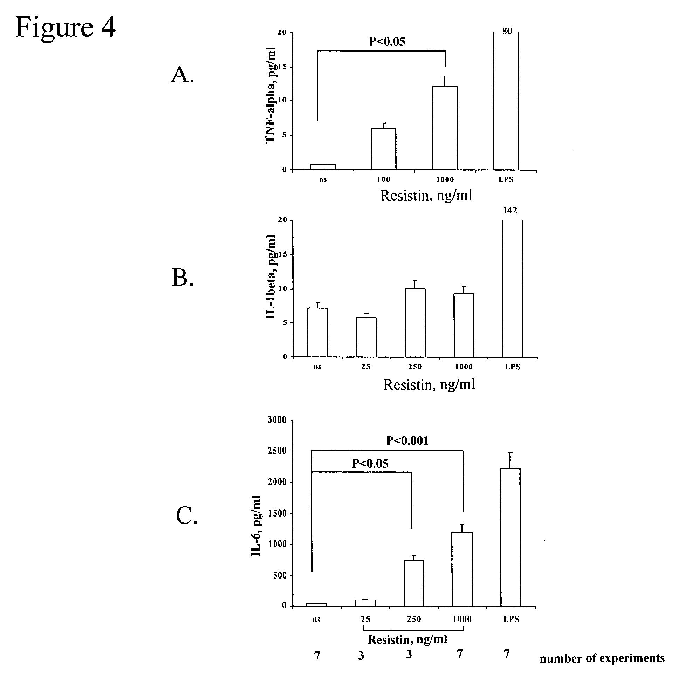

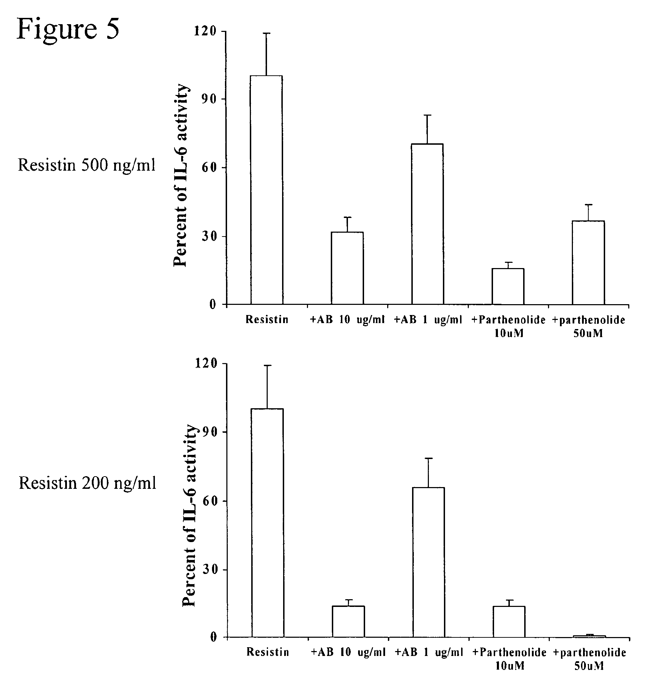

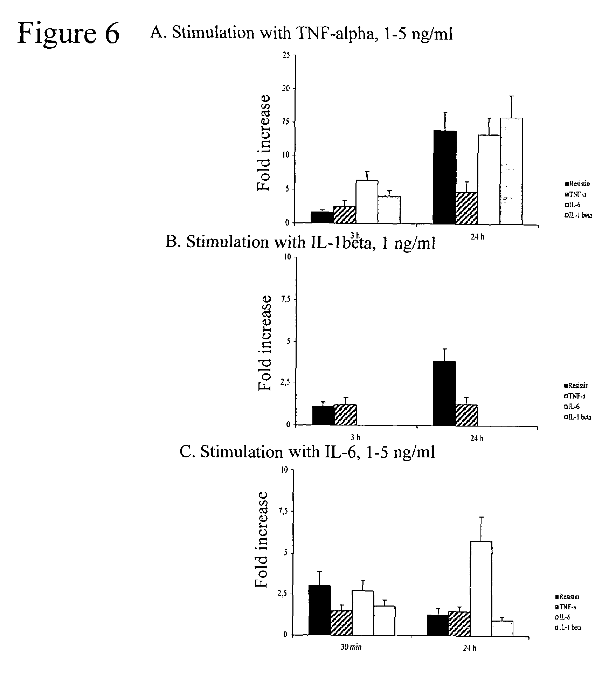

To assess the role of resistin in the induction of inflammation, PBMC were stimulated with increasing concentrations of resistin (10-1000 ng/ml). The analyses of cell lysates following resistin stimulation demonstrated an induction of genes for proinflammatory cytokines (TNF-alpha, IL-6, IL-1b) (Fig. 3a,b). mRNA for TNF-alpha and IL-6 were the early findings, detectable already after 30 min of resistin stimulation. IL-6 mRNA continued to increase following 24 h of resistin stimulation throughout the experiment, while TNF-alpha mRNA levels declined after 3 h. We also observed that stimulation with resistin led to self-stimulation of resistin gene in PBMC providing a positive feedback mechanism. Increase of resistin mRNA was detectable following 3 h and proceeded up to 24 h of the experiment. Findings on mRNA level were also supported by the analysis of cytokine release into supernatants following 48 hours of stimulation with resistin. A dose dependent increase of IL-6 activity and the levels of TNF-alpha and IL-1b were found in all PBMC cultures (Fig. 4a,b,c). Preincubation of recombinant resistin with monoclonal anti-resistin antibodies (final concentration 1-10 µg/ml) prior to stimulation of PBMC cultures resulted in a dose-dependent down-regulation of IL-6 release. Pronounced reduction of IL-6 activity by 34% was observed already with the lowest concentration of antibodies, and effect continued to increase reaching 77% (range 68-86%) with the highest concentration of antibodies used (Fig.5). Stimulation of human PBMC (n=5) with proinflammatory cytokines (TNF-alpha, IL-6, IL-1b) at physiological concentrations showed that only TNF-alpha (final concentration 1-5 ng/ml) could induce the expression of resistin giving rise to resistin mRNA, which continuously increased during the whole experimental period (Fig.6). Relevance of TNF-alpha dose chosen for PBMC stimulation was proved by the pattern of IL-6 and IL-1b mRNA that were detected simultaneously with resistin mRNA. Stimulation with IL-6 and IL-1b was less efficient with respect to induction of resistin mRNA and declined by 24 h. Stimulation of human monocytic cell line THP-1 with resistin resulted in cytokine production in the pattern similar to PBMC (not shown), while neither stimulation with TNF-α nor with LPS gave rise to the expression of resistin gene. In vitro stimulation of synovial cells from patients with acute synovitis (n=4) with resistin resulted also in an increased expression of TNF-alpha and IL-6 genes in pattern similar to human PBMC. The latter observation suggests that monocytic cells are possible target cells for resistin within synovial tissue.

### The role of resistin in the intracellular signaling

To assess the possible mechanism for cytokine induction we studied the ability of resistin to activate NF-kappaB and AP-1 signalling pathways. Nuclear extracts of PBMC stimulated with resistin (0-1000 ng/ml) were subjected to EMSA.

To further assess the role of NF-kappaB in resistin-induced cytokine activation, a specific NF-kappaB inhibitor, parthenolide, was introduced in the PBMC culture prior to resistin stimulation. The evaluation of supernatants following 48 h of stimulation with resistin revealed a suppression of IL-6 activity in the cultures treated with parthenolide (10 and 25 µM) by 85% (range 84-88%) as compared to non-treated cultures (Fig.5). Stimulation of PBMC with LPS (10 ng/ml) resulted in upregulation of resistin gene and in an increase of extracellular resistin in supernatants following 48 hours of stimulation. Level of IL-6 in this supernatants was also significantly elevated. Introduction of NF-kappaB inhibitor parthenolide in the PBMC culture prior to LPS stimulation led to a reduction of resistin level as compared to parthenolide non-treated cell cultures.

## Claims

1. Use of an agent blocking the activity of resistin for the preparation of a medicament for the treatment of rheumatoid arthritis.

2. The use according to claim 1, wherein the agent is a compound preventing transcription and/or translation of resistin.

3. The use according to claim 2, wherein the compound is a nucleotide sequence antisense to the resistin mRNA or parts thereof, or PPAR-gamma.

4. The use according to claim 1, wherein the agent is a compound blocking protein activity.

5. The use according to claim 4, wherein the compound is an anti-resistin antibody, thiazolidinedione, oxazolidinedione, oxadiazolidinedione derivatives, glitazones, PPAR alfa agonists, such as leukotriene B4 and 8(S)-hydroxyeicosatetraenoic acid, LXR agonists, such as Acetyl-podocarpic dimer (APD), or mixtures thereof.

6. The use according to any of the preceding claims, wherein the treatment comprises preventing or alleviating the symptoms associated with Rheumatoid Arthritis.

7. The use according to any of the preceding claims, wherein the agent is administered topically or systemically.

8. The use according to claim 7, wherein the agent is formulated as a solution, suspension, emulsion, a powder, dragee, tablet, capsule, spray, aerosol, cream, gel, ointment, spray, aerosol, or patch.

9. A method for diagnosing the onset of Rheumatoid arthritis in a individual comprising the steps of :
(i) determining the level of resistin in a sample of a patient deemed to have developed Rheumatoid Arthritis; and
(ii) comparing the level with a control, obtained from a healthy individual;
wherein an increase of the level of resistin is indicative of the onset of Rheumatoid Arthritis.
